# EUROPEAN PATENT APPLICATION

(11) **EP 0 981 050 A1**
(43) Date of publication of application: **23.02.2000**
(21) Application number: 98202732.8
(22) Date of filing: 14.08.1998
(51) Int. Cl.: G01N 33/68, G01N 33/569

(54) **Immunoassay for hazelnut allergens**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Koppelman, Stef J., 3723 DP De Bilt (NL); Van Duijn, Gert, 3514 XK Utrecht (NL); Hessing, Martin, 3994 ED Houten (NL)
(74) Representative: de Bruijn, Leendert C.

(57) **Abstract**

A method and a test kit for determining immunochemically reactive components of hazelnut allergens is described. The method uses one or more antihazelnut antibodies in an ELISA to improve food safety with respect to hazelnut allergy. The antibodies are produced by:
- isolating hazelnut protein from a hazelnut sample;
- checking the isolated protein for the presence of allergens;
- raising and isolating antibodies against the checked protein;
- immunopurifying the isolated antibodies.

## Description

### Field of the invention

The present invention relates to methods for detecting and/or determining the concentration of allergenic substances of hazelnuts in fluids such as extracts of foods, hazelnut extracts for diagnostic use, and (hydrolysates of) hazelnut extracts for therapeutic purposes. In another aspect it relates to immunometric and inhibition assay techniques. In yet another aspect it relates to polyclonal anti-hazelnut protein antibodies. Furthermore, this inventions relates to methods of preparing such an extract, such antibodies, and test kits constructed with these compounds.

### Background

The determination of the presence or concentration of antigenic or allergenic substances, for example those related with a wide variety of physiological disorders, as food allergies, in extracts, serum (or other body fluids) or preparations for diagnostic or therapeutic use relies increasingly upon immunoassay techniques. These techniques are based upon formation of a complex between the antigenic substance being assayed and an antibody or antibodies in which one or the other member of the complex may be labelled, which permits its detection and/or quantitative analysis after separation of the complexed labelled antigen or antibody from uncomplexed labelled antigen or antibody.

Hazelnut allergy affects a substantial part of the Western populations and often coincides with allergies to tree pollen [Andersen, K.E., Lowenstein, H. *Contact Dermatitis* **4**, 73-79, **1978**; Lowenstein, H., Eriksson, N.E. *Allergy* **38**, 577-587, **1983**]. Since treatment of hazelnut allergy is not yet available, patients need to exclude hazelnuts from their diets. Hazelnuts are used in confectionery products like nougat and in chocolate products. Recent product diversification led to an increase in consumer products containing hazelnuts. Many of these new products, like muesli-bars and chocolate spreads, are produced in facilities were products that normally do not contain hazelnuts are formulated too. This increases the risk on cross-contamination with hazelnut and with the growing interest in food allergy, the food industry is confronted with this problem. Although there is no legislation on labelling of food allergens on the ingredient list [Smith, J. *International Food Ingredients*, 13-14, **1997**], most producers and retailers try to minimise the risk on cross-contamination. However, until now the food industry has only limited access to tests for the detection for hazelnuts, in contrast with for example peanut tests [Koppelman, S.J., Bleeker Marcelis, H., Duijn, G.-v., Hessing, M. *World of Ingredients*, 35-38, **1996** (December); Yeung, J.M., Collins, P.G. *J AOAC Int*. **79**, 1411-1416, **1996**].

A limited number of immunochemical tests for the detection of hazelnuts in foods have been described [Mohr, E., Wichmann, Penkala, M., Winoto, S. *Gordian*. **83**, 193-194, **1983**; Klein, E., Baudner, S., Guenther, H.O.. *Zeitschrift fuer Lebensmittel Untersuchung und Forschung* **180**, 30-35, **1985**; Klein, E., Guenther, H.O. *Z. Lebensm*. *Unters. Forschung* **180**, 36-40, **1985**; Gunther, H.O. *Ernaehrung* **10**, 91-94, **1986**; Garrone, W., Antonucci, M., Bona, U., Clementi, S. *Lebensmittel Wissenschaft und Technologie* **21**, 76-82, **1988**]. These tests were developed mainly for the quantification of hazelnuts in hazelnut-containing products with respect to product adulteration and therefore special attention was paid to specificity and reproducibility. Also, the effect of roasting on the recognition in these tests was investigated. Because hazelnut-containing products like chocolate and confectionery products contain substantial amounts of hazelnut, usually between 1 and 15%, the lower detection limit was relatively high, in the order of 100 ppm [Klein, E., Baudner, S., Guenther, H.O. *Z. Lebensm. Unters*. *Forschung*. **180**, 30-35, **1985**]. No assays were described for the detection of minor amounts of hazelnut proteins which are important in the provocation of allergic reactions.

Little is known about the exact levels of hazelnuts leading to a reaction in allergic individuals, and most likely, these levels will vary between patients. Taylor postulated that immuno-assays for the detection of food allergens should have detection limits of at least 10 ppm [Taylor, S.L., Nordlee, J.A. *Food Technology*. 231-238, **1995**]. The aim of this study was to compare different immunological assays in their ability to detect and quantify hazelnut protein in foods with a high sensitivity and specificity. We used both patient IgE antibodies and polyclonal IgG antibodies from rabbits sensitised with hazelnut and we developed an assay with a high sensitivity and specificity, potentially applicable for the food industry.

Hazelnuts are widely used in the food industry owing to their nutritive value and taste. The amount of hazelnut present in a recipe is usually considered as a quality mark. However, contamination of foods that normally do not contain hazelnuts is a threat for patients with a hazelnut allergy. For this reason, the availability of a method for the detection and quantification of hazelnuts in foods is desired.

### Description of the invention

The present invention relates to a method and a test kit for the detection and quantification of minor amounts of hazelnut allergens in food products as defined in the appending claims. These allergens can be determined whether or not other hazelnut components are present. Several immunochemical methods e.g. Western blotting and elisa, were constructed with both patient sera and sera of rabbits hypersensitised with hazelnut protein as a source of antibodies. These methods were tested on their ability to detect and quantify hazelnuts in foods. Western-blotting appeared to be a non-specific method when patient sera were used as a source of antibodies. Using immuno-purified antibodies from rabbits sensitised to hazelnuts, Western-blotting became specific, but the sensitivity of this method was limited. Inhibition of IgE binding is a generally used test in clinical laboratories to establish contamination with hazelnuts of complaint samples. This approach is sensitive and specific, but not well accessible for the food industry since patient serum is needed. A sandwich elisa constructed with a immuno-purified antibody from rabbits sensitised to hazelnuts was found to be both specific and sensitive. No substantial cross-reactivity with other nuts, legumes or other food constituents was observed, and concentrations as low as 5 ng/ml, corresponding to 1 ppm in food products, were detected. In a field test, several complaint samples were shown to contain traces of hazelnut. Thus, the sandwich elisa constructed with immuno-purified for rabbits sensitised with hazelnut protein is a sensitive and specific method to detect and quantify hazelnut protein in consumer products. Since this test does not require patient serum as a reagent, it is potentially applicable for the food industry and may contribute to safer consumer products. An inhibition (competition) elisa using immuno-purified rabbit or other mammalian antibodies is equally suitable as is it is both selective and sensitive.

Mammalian antibodies for use in the elisa methods of the invention may be derived from any suitable mammal, such as goat, rabbit, rat, mouse etc. The limit of detection of the method of the invention is 10 ppm or lower, and may be as low as 1 ppm or even 0.1 ppm, in the sample to be tested for the presence of hazelnut allergens. The method is suitable for determining hazelnut allergens over a broad concentration range, up to 1000 ppm or even 1%.

Different immunochemical methods for the detection and quantification of hazelnut protein in food products were compared. Our primary goal was to reach a high sensitivity (10 ppm or better) in order to detect minor amounts of hazelnut protein as they play a role in the provocation of reactions in patients with a hazelnut allergy. First we used serum from 4 patients with a hazelnut allergy as a source for antibodies against hazelnuts. Hazelnuts were detected although the sensitivity was poor (500 µg/ml, corresponding to 1% or 10.000 ppm in a food product). Peanuts, soya, hen's eggs and milk cross-reacted substantially in this test. These raw materials were tested on cross reactivity because of they are common protein sources for products potentially contaminated with hazelnuts. The observed cross-reactivity can be explained in two ways. First, since allergy to hazelnuts often coincides with other (food) allergies [Andersen, K.E., Lowenstein, H. *Contact Dermatitis.* **4**, 73-79, **1978**; Lowenstein, H., Eriksson, N.E. *Allergy*. **38**, 577-587, **1983**], the serum of the patients may contain IgE directed to tested proteins. Alternatively, epitopes on the tested proteins may resemble allergic epitopes as found on hazelnut proteins. The denaturing character of the Western blotting procedure by the use of SDS contributes to the exposition of epitopes that are buried at the interior of the protein under native conditions, thereby increasing the possibility on epitope cross-reactivity [Towbin, H.K., Staehelin, T.H., Gordon, J. *Proc. Natl. Acad. Sci. USA.* **76**, 4350-4354, **1979**]. To exclude cross-reaction based on the presence of IgE directed to other proteins, inhibition of IgE binding to immobilised hazelnut protein was studied. In this set-up only IgE directed to hazelnut protein plays a role. IgE that cross-reacts with other components due to epitope similarity can still occur. This approach results in a very high specificity; only walnuts and to a lesser extent cashew nuts and pecan nuts were recognised. This cross-reactivity was 200 times less then the reactivity of hazelnuts. The cross-reacting samples are phylogenetically related, indicative for similarity of epitopes. Hazelnut allergy often coincides with peanut and apple allergy. These allergens did not give rise to cross-reaction in this assay, indicating that, in contrast with the Western-blotting procedure, IgE directed to other proteins than from hazelnut are excluded in this experimental set up. The high specificity allows the use of relative high concentrations of patient serum, resulting in a high sensitivity. Concentrations as low as 10 ng/ml, corresponding with 0.2 ppm in food products, were detected, indicating that this assay is applicable for the detection of minute traces of hazelnuts. A drawback for the food industry for application of this test is the use of patient serum. Therefore, rabbits were sensitised with hazelnut protein in order to obtain polyclonal rabbit antibodies directed against hazelnuts. Immuno-purification was applied to improve the specificity of these antibodies. The immuno-purified antibodies were used in Western-blotting resulting in a reasonable specificity compared to the Western-blotting using patient serum, indicating that in the latter approach the presence of IgE against other components indeed caused the major cross-reactivity. Application of the rabbit antibodies on Western blotting led to a sensitivity of 50 µg/ml, corresponding with 0.1% or 1000 ppm in food products. Attempts to improve the sensitivity by means of increasing the concentration of rabbit antibodies or the incubation times led to a higher cross-reactivity and higher backgrounds, respectively, and were therefore not applicable. A sandwich elisa constructed with the rabbit antibodies showed a specificity comparable with the assay based on inhibition of IgE binding as illustrated by the fact that only walnuts cross-reacted. This cross-reactivity was more than 1300 times less than the reactivity of hazelnuts, indicating that this assay is well applicable for the detection of hazelnuts in miscellaneous consumer products. The sensitivity of this assay was 0.1 ppm which is considered to be safe [Taylor, S.L., Nordlee, J.A. *Food Technology.* 231-238, **1995**], and is in the same order of magnitude as for a sandwich elisa developed earlier for the detection of peanut proteins [Koppelman, S.J., Bleeker Marcelis, H., Duijn, G.-v., Hessing, M. *World of Ingredients* 35-38, **1996** (December)]. Furthermore, we show that roasted hazelnuts were recognised to the same extent, which is a requirement since roasted hazelnuts are usually applied in recipes for consumer products.

In this study we have compared different current approaches to detect hazelnuts in foods. In contrast to earlier described tests, we paid special attention to obtain a high sensitivity. We found that a sandwich elisa constructed with immuno-purified antibodies obtained from rabbits sensitised with hazelnut protein was able to detect traces of hazelnut as low as 0.1 ppm. The high specificity allows to apply this assay for miscellaneous consumer products. The assay does not contain patient serum and is therefore potentially applicable for the food industry. The availability of test for the detection of traces of food allergens [Koppelman, S.J., Bleeker Marcelis, H., Duijn, G.-v., Hessing, M. *World of. Ingredients* 35-38, **1996** (December); Yeung, J.M., Collins, P.G. *J AOAC Int*. **79**, 1411-1416, **1996**] and the growing interest in food allergy, both under clinicians and the food industry, will lead to safer consumer products in the future.

### Example 1: Hazelnut protein extracts and food extracts

Hazelnut protein extracts were made by mixing 1 gram ground hazelnut with 20 ml 20 mM Tris buffer (pH 8.2). After 2 hours stirring at room temperature, the aqueous layer was collected by centrifugation (3.000 g, at room temperature). The aqueous phase was subsequently centrifuged (10.000 g at room temperature) to remove residual traces of fat and insoluble particles. The clear extracts were extensively dialysed against 20 mM Tris buffer (pH 8.2) at 4°C. Protein concentrations were determined using Bradford analysis with BSA as a standard. SDS-PAGE from extracts from ground hazelnuts showed a similar pattern as described by Hirschwehr *et al.* [Hirschwehr, R., Valenta, R., Ebner, C., Ferreira, F., Sperr, W.R., Valent, P., Rohac, M., Rumpold, H., Scheiner, O., Kraft, D. *J. Allergy Clin. Immunol.* **90**, 927-936, **1992**]. Hazelnut extracts usually contained 5 mg/ml protein as a yield of 1 gram solids in 20 ml. Therefore, in calculations between hazelnut protein concentrations in extracts (mg/ml) and hazelnuts in food products (g/g), a factor of 0.2 is used. The amounts of hazelnut in food products are subsequently expressed as percentage or in ppm if more appropriate (1% corresponds with 10.000 ppm). Food extracts were made by mixing 1 gram homogenised sample with 20 ml 20 mM Tris buffer (pH 8.2). After 2 hours stirring at room temperature, the aqueous layer was collected by centrifugation (3.000 g, at room temperature). The aqueous phase was subsequently centrifuged (10.000 g at room temperature) to remove residual traces of fat and insoluble particles. Extracts were stored at -20°C.

### Example 2: Preparation and characterisation of rabbit antibodies directed against hazelnut protein

Serum from two rabbits sensitised i.p. with a crude hazelnut protein extract was collected and tested in a direct for hazelnut protein binding in a direct elisa. Titres were estimated on 10⁵ to 10⁶ in a direct elisa. Specific IgG was isolated using immuno-affinity chromatography. Hazelnut protein as present in a hazelnut protein extract (see above) was covalently coupled to CNBr-activated Sepharose according to the manufacturers instructions (final concentration 1 mg protein per ml of swelled Sepharose). Serum was applied on the hazelnut protein-column (10 ml serum per 10 ml column volume) in PBS. The column was washed with PBS until the OD₂₈₀ of the flowthrough was less then 0.01. The bound fraction was eluted with 0.1 M Glycine (pH = 2.7) and fractions were immediately neutralised with a 10% volume of 1 M Tris, pH 9.0. Protein containing fractions were pooled, dialysed against PBS and stored at -20°C until use. On SDS-PAGE, one main band was present at approximately 150 kDa and under reducing conditions two bands were present at approximately 50 kDa and 25 kDa indicating that the preparation consists mainly of IgG. Part of these immuno-purified antibodies were covalently conjugated to horse radish peroxidase according to the manufacturers instructions and stored in 50% glycerol at -20°C until use.

### Example 3: Western-blotting using patient serum or immuno-purified antibodies from rabbit sensitised with hazelnuts

SDS-PAGE was performed essentially according to Laemlli [Laemmli, U.K. *Nature.* **227**, 359-365, **1970**] using a BioRad Mini Protean II system (BioRad) with 15 % acrylamide gels (15 x 10cm). Pre-stained molecular weight markers with molecular weights of 14.3, 21.5, 30, 46, 66, 97.4 and 220 kDa were used as reference. Samples were mixed in a 1:1 ratio with 63 mM TRIS buffer (pH 6.8) containing 1% DTT, 2% SDS, 0.01% bromophenol blue and 20% (v/v) glycerol and were subsequently boiled for 5 minutes. The final protein concentration of the samples applied on the gel (20 µl) was 500 µg/ml unless otherwise stated. Gels were stained with Coomassie Briliant Blue R-250 dissolved in de-staining solution (10% HAc (v/v), 5% methanol (v/v) in water). After de-staining, gels were scanned with an ImageMaster DTS (Pharmacia, Uppsalla, SE). To study the immuno-reactivity of the proteins, SDS-PAGE gels were prepared as described above and the separated proteins were transferred to polyvinyldifluoride sheets (Immobilon-P, Millipore Corp. Bedford, MA) generally as described by Towbin et al. [*Proc. Natl. Acad. Sci. USA.* **76**, 4350-4354, **1979**]. Membranes were blocked with 3% BSA in wash buffer (50 mM TRIS, pH 7.5, containing 0.1% BSA and 0.1% Tween 20) for 1 hour at room temperature. A pool of serum of patients with a hazelnut allergy diluted 1/10 in wash buffer, or a peroxidase-conjugate of immuno-purified antibodies from rabbits sensitised with hazelnut were applied on the membrane (overnight at room temperature). Bound IgE was detected using a commercial anti-human IgE conjugated to peroxidase, whereas bound peroxidase-conjugate of immuno-purified antibodies from rabbits sensitised with hazelnut was detected directely. Conjugates were subsequently detected by means of astaining reaction for peroxidase activity. Between each step, blot membranes were washed thoroughly with wash buffer for 5 times. Blots were dried and scanned with an ImageMaster DTS (Pharmacia, Uppsala, SE). Immuno-reactivity was quantified by calculating the mathematical product of the intensity and surface of specific hazelnut protein bands using known amounts of hazelnut protein as a standard. Non-specific binding of the anti-IgE antibody conjugate to proteins on the membrane was measured in a similar blotting procedure omitting the incubation with patient sera, and appeared to be neglectible. The pattern observed using patient serum shows bands as observed for hazelnuts on SDS-PAGE stained with Coomassie, indicating that distinct hazelnut proteins bind IgE. The sensitivity is poor since samples containing less than 50 µg/ml hazelnut protein are not clearly visible. Taking into account the protein level of the hazelnut extract, this threshold corresponds with a hazelnut concentration of 1% food products. Peanut, soya, milk and hens eggs cross-react significantly. The low sensitivity and the cross-reactivity of other food proteins indicated that a major part of the IgE present in the patient serum was directed to other proteins than from hazelnut. As to the reactivity towards immuno-purified rabbit antibodies from rabbits sensitised with hazelnut, concentrations between and 15 µg/ml and 500 µg/ml were detected in a dose dependent way, corresponding to 0.3 and 10% hazelnuts in food products respectively. The most intense band at about 55 kDa was quantified for all samples using a gel scanner and expressed as the mathematical product of intensity and surface of each band. A calibration curve was fitted with high accuracy. When studying cross-reactivity of several food constituents, faint bands are visible for peanut and soy, whereas milk and hens eggs do not exhibit any cross-reaction. For products with a known recipe, excluding the cross-reacting proteins, this assay was applied successfully. Since the band pattern for hazelnut is clearly different from that of soya and peanut (main bands at 25 and 30 kDa respectively), recipes containing limited amounts of these legumes could be analysed as well, although the interpretation requires great care. Hazelnut was detected in both complaint samples obtained from patients with a hazelnut allergy and in products with an increased change for contamination with traces of hazelnut.

### Example 4: IgE inhibition Elisa

Dilutions of extracts of hazelnuts or food products were incubated in a 1 to 30 dilution of patient serum in PBS containing 1% BSA and 0.1% Tween 20. In this fluid phase, hazelnut proteins were allowed to bind to IgE for one hour at room temperature. In order to determine the unbound IgE fraction, the incubation mixtures were transferred to the 96-well plates previously treated as follows. 96-well plates were coated with 10 µg/ml hazelnut protein in PBS and then blocked with BSA (1%) in PBS containing 0.1% Tween 20. IgE bound to the wells coated with hazelnut proteins was detected using an anti-human IgE antibody conjugated to horse radish peroxidase. Peroxidase activity was detected with an ortho-phenylenediamine staining procedure. Between each step, plates were washed five times with PBS containing 0.1% Tween 20. Extracts of native, not heat-treated hazelnuts were used as calibrator. Hazelnut allergen-dependent inhibition of IgE binding to plates coated with hazelnut protein was observed in a sensitive range from 10 ng/ml to 1 µg/ml, indicating that 2 ppm hazelnut in food products is recognised with this test. Roasted hazelnuts inhibit IgE binding too, although somewhat less potently. An advantage of this assay compared to Western-blotting is that only IgE directed to hazelnut proteins plays a role. Therefore, the specificity is increased compared to the Western-blotting method. Extracts of common protein-rich food constituents were analysed in the inhibition elisa. It was found that walnuts, and to a lesser extend cashew and pecan nuts, give rise to inhibition of IgE binding. This may be caused by true cross-reaction on IgE level. Alternatively, matrix effects from the tested sample may also the results. Taking into account the dilution factors of the extracts, the cross-reactivity with walnuts is over 200 times less than the reactivity of hazelnuts.

### Example 5: Sandwich Elisa

A sandwich Elisa was constructed as follows. Elisa plates were coated with 1 µg/ml immuno-purified anti-hazelnut IgG (overnight at room temperature). Plates were blocked with BSA (1%) in PBS containing 0.1% Tween 20. Samples were diluted in PBS containing 1% BSA and 0.1% Tween 20 and subsequently incubated for 2 hours at room temperature. After washing, plates were incubated with peroxidase conjugated immuno-purified anti-hazelnut IgG conjugated to peroxidase and bound peroxidase activity was detected with an ortho-phenylenediamine staining procedure. Between each step, plates were washed five times with PBS containing 0.1% Tween 20. Extracts of native, not heat-treated hazelnuts were used as calibrator. The sensitive range of this assay was from 5 ng/ml to 1µg/ml, which is in the same order of magnitude as found for the IgE inhibition test. Extracts of roasted hazelnuts shows a similar calibration curve, indicating that this assay is able to detect roasted hazelnuts as well. Since this test is most promising for application in the food industry, an extensive study of possible cross-reactions was performed. Only walnut showed a significant cross-reaction, although it reacts approximately 1300 times less than hazelnut. Cashew nut, almond and brazil nut show a detectable, but minor, cross-reaction.

The results of determinations of the presence of hazelnut allergens in some commercial products using the method of this example ar summarized in table 1.

**Table 1**

| Product | Type | Manufacturer | Hazelnut | |
|---|---|---|---|---|
| | | | declared | measured (ppm) |
| chocolate spread | natural | A | - | 752 ± 59 |
| | hazelnut | A | 13 % | 71000 ± 19000 |
| | banana | A | - | 115 ± 15 |
| | milk flavour | A | - | 11 ± 2 |
| | hazelnut | B | + | 2.5 ± 0.5 |
| chocolate bar | caramel | C | - | < |
| | milk | D | - | 3.4 ± 0.4 |
| | hazelnut | D | + | 135000 ± 5000 |
| | natural | E | - | 14 ± 0.4 |
| | hazelnut | E | 25 % | 230000 ± 10000 |
| chocolate cookie | hazelnut | A | 5 % | 50000 ± 7000 |
| | whole wheat | A | - | 6.2 ± 0.2 |
| | natural | A | - | 557 ± 74 |
| | haazelnut | G | 5 % | 50000 ± 4000 |
| | natural | G | - | 18 ± 1.4 |
| muesli cookie | nut | J | | 11000 ± 1000 |
| | apple | J | - | 5.0 ± 0.2 |
| | fruit | J | - | 27 ± 1.2 |
| cake | ginger | K | - | < |
| | ginger/nuts | M | + | 38000 ± 6000 |
| | caramel | O | - | < |
| complaint sample chocolate spread | milk flavour | B | - | 4000 ± 1000 |
| < means below 1 ppm | | | | |

## Claims

1. An immunochemical method of determining the presence of one or more hazelnut allergens or parts thereof in a test sample, characterised by using labeled antibodies to hazelnut protein, which antibodies have been produced by:
- isolating hazelnut protein from a hazelnut sample;
- checking the isolated protein for the presence of allergens;
- raising and isolating antibodies against the checked protein;
- immunopurifying the isolated antibodies.

2. A method as claimed in claim 1, characterised by using a sandwich ELISA.

3. A method as claimed in claim 1, characterised by using a competition ELISA.

4. A method as claimed in any one of claims 1-3, characterised by determining the presence of hazelnut allergens present in the test sample at a level of 100 ppm or lower.

5. A method as claimed in claim 4, characterised by determining the presence of hazelnut allergens present in the test sample at a level of 10 ppm or lower.

6. Test kit for determining the presence of hazelnut allergens in a test sample, comprising polyclonal antibodies to hazelnut protein which have been produced by:
- isolating hazelnut protein from a hazelnut sample;
- checking the isolated protein for the presence of allergens;
- raising and isolating antibodies against the checked protein;
- immunopurifying the isolated antibodies.

7. Test kit as claimed in claim 6, comprising immobilised anti-hazelnut antibodies and labeled anti-hazelnut antibodies, for carrying out a sandwich ELISA.

8. Test kit as claimed in claim 6, further comprising immobilised hazelnut protein and labeled antibodies to said anti-hazelnut antibodies, for carrying out a competition ELISA.
